# EUROPÄISCHE PATENTSCHRIFT

(11) **EP 2 231 084 B1**
(45) Veröffentlichungstag und Bekanntmachung des Hinweises auf die Patenterteilung: **07.09.2011**
(21) Anmeldenummer: 09702311.3
(22) Anmeldetag: 16.01.2009
(51) Int. Cl.: A61F 9/008, A61F 9/011, B23K 26/06

(54) **LASERKORREKTUR VON SEHFEHLERN AN DER NATÜRLICHEN AUGENLINSE**
LASER CORRECTION OF VISION CONDITIONS ON THE NATURAL EYE LENS
CORRECTION LASER DE DÉFAUTS VISUELS SUR UNE LENTILLE OCULAIRE NATURELLE

(30) Priorität: 18.01.2008 DE 102008005053
(43) Veröffentlichungstag der Anmeldung: 29.09.2010
(73) Patentinhaber: Rowiak GmbH, 30419 Hannover (DE)
(72) Erfinder: LUBATSCHOWSKI, Holger, 30989 Gehrden (DE); OBERHEIDE, UWE, 50674 Köln (DE); SCHUMACHER, Silvia, 30451 Hannover (DE)
(74) Vertreter: Grünecker, Kinkeldey, Stockmair & Schwanhäusser Anwaltssozietät
(86) Internationale Anmeldenummer: PCT/EP2009/000274
(87) Internationale Veröffentlichungsnummer: WO 2009/090095

(56) Entgegenhaltungen:
- WO-A1-2005/070358
- WO-A2-93/08677
- DE-A1-102004 033 819
- US-A1- 2005 182 489
- YAMADA KAZUHIRO ET AL: "Multilevel phase-type diffractive lenses in silica glass induced by filamentation of femtosecond laser pulses" OPTICS LETTERS, Bd. 29, Nr. 16, 15. August 2004 (2004-08-15), Seiten 1846-1848, XP002526489 OPTICAL SOCIETY OF AMERICA ISSN: 0146-9592

## Beschreibung

Die vorliegende Erfindung bezieht sich auf ein neuartiges Lasersystem und Verfahren zur Korrektur von Sehfehlem wie Weitsichtigkeit (Hyperopie), Kurzsichtigkeit (Myopie), Astigmatismus oder Alterssichtigkeit (Presbyopie). Das erfindungsgemäße Lasersystem und Verfahren sehen vor, dass die Sehfehlerkorrektur durch eine Behandlung oder Bearbeitung der natürlichen Augenlinse eines Patienten erfolgt.

Ultrakurze Laserpulse mit einer Dauer im Bereich einiger Femtosekunden (fs) bis zu Pikosekunden (ps) sind dafür bekannt, mittels des sogenannten optischen Durchbruchs Disruptionen in oder auf transparenten Medien zu erzeugen. Die Disruption führt zu einem Abtrag bzw. zu einem Wegreißen von Material. Der Wechselwirkungsprozess basiert auf Multiphotonenabsorption und ist in einer Vielzahl von Publikationen bereits beschrieben worden (siehe beispielsweise Alfred Vogel and Vasan Venugopalan: "Mechanisms of Pulsed Laser Ablation of Biological Tissues"; Chem. Rev. 2003, 103, 577-644; oder die US Patente Nr. US 5,656,186 A oder US 5,984,916 A). Charakteristisch ist zum Einen, dass die vom Laser erzeugte Disruption lokal sehr begrenzt ist, und zum Andern, dass in für die Laserstrahlung transparenten Materialien der Ort der Disruption frei in drei Dimensionen platziert werden kann.

Die US 6,552,301 B2 befasst sich ausführlich mit dem Bohren von Löchern mittels ultrakurzer Laserpulse. Nur nebenbei wird erwähnt, dass auch im Mateiralinneren gearbeitet werden kann. Lediglich in äußerst knapper Form und ohne die Angabe irgendwelcher Details wird angedeutet, dass ultrakurze Laserpulse auch für photorefraktive Chirurgie genutzt werden können.

In der Augenheilkunde (Ophthalmologie) wird der Materialabtrag mittels optischen Durchbruchs auf dem Gebiet der refraktiven Chirurgie genutzt, d.h. für Eingriffe und-Operationen zur Korrektur der Brechkraft des Auges. Die DE 199 38 203 A1 und die dazu nahezu inhaltsgleiche DE 100 24 080 A1 beschreiben in recht allgemeinen Worten mehrere unterschiedliche Verfahren, insbesondere die Umformung der Hornhaut (Komea) durch Materialabtrag mittels gepulster Laser, unter anderem durch Ultrakurzpulslaser. Auch die DE 10 2004 033 819 A1 beschreibt u.a. Verfahren zur refraktiven Chirurgie mit fs-Pulsen. Zur Behandlung von Presbyopie schlägt die WO 2005/070358 A1 vor, durch Materialabtrag mittels fs-Laserpulsen in die Oberfläche der natürlichen Augenlinse Schnitte zu setzen, um die Elastizität der Augenlinse und damit deren Akkommodationsfähigkeit zu vergrößern.

Weitere Untersuchungen zu den Auswirkungen der Photo-Disruption in der refraktiven Chirurgie der Komea des Auges finden sich in Kurtz RM, Horvath C, Liu HH, et al.: "Lamellar refractive surgery with scanned intrastromal picosecond and femtosecond laser pulses in animal eyes", J Refract Surg. 1998;14:541-548; oder in R. Krueger, J. Kuszak, H. Lubatschowski et al.: "First safety study of femtosecond laser photodisruption in animal lenses: Tissue morphology and cataractogenesis", Journal of Cataract & Refractive Surgery, Volume 31, Issue 12, Pages 2386-2394. Dabei hat sich in der Hornhaut des Auges gezeigt, dass Veränderungen, die innerhalb des kornealen Stromas bei moderater Laserenergie hervorgerufen werden, beispielsweise um für die LASIK-Operation einen sogenannten kornealen Flap zu schneiden, innerhalb weniger Tage bis Wochen völlig ausheilen und keine sichtbaren Veränderungen hinterlassen [Heisterkamp A, Thanongsak M, Kermani O, Drommer W, Welling H, Ertmer W, Lubatschowski H: "Intrastromal refractive surgery with ultrashort laser pulses - in vivo study on rabbit eyes"; Graefes Archives of Clinical and Experimental Ophthalmology 241 (6),511-517 (2003)]. Zumindest wird das durchtretende Licht nicht soweit beeinflusst, dass die behandelten Patienten eine Störung erfahren.

Je geringer die verwendete Pulsenergie und je stärker die Fokussierung (d.h. je höher die Numerische Apertur, NA, der Fokussieroptik), umso präziser, d.h. in seiner Ausdehnung kleiner, ist die laserinduzierte Disruption und der damit erzielte Materialabtrag. Bei dem optischen Durchbruch handelt es sich jedoch um einen Schwellenprozess. Es existiert in Abhängigkeit vom Material des Werkstücks eine auch als "Abtragsschwelle" oder "Disruptionsschwelle" bezeichnete Schwelle (angegeben in Intensität oder Energie pro Fläche, d.h. Fluenz), unterhalb derer keine Disruption und kein Materialabtrag mehr stattfindet.

Auch unterhalb der Disruptionsschwelle kann jedoch noch eine Veränderung der Materialeigenschaften des Werkstücks auftreten. Sie kann in einer chemischen Veränderung bestehen, die von freien Elektronen verursacht wurde, die durch Multiphotonenabsorption oder vergleichbare, laserinduzierte Ionisationsprozesse entstanden sind. Es können auch photochemische Veränderungen sein, die beispielsweise durch nichtlineare Erzeugung von blauem oder UV-Licht verursacht wurden. Erst bei höheren Energien treten photothermisch induzierte oder plasmainduzierte lokale Zerreißungen des Mediums auf. Die Veränderung der Materialeigenschaften kann z.B. in einem lokal begrenzten Verschmelzen bestehen, so dass sich das Material lokal zusammenzieht. Möglich ist auch das lokal begrenzte Verändern des Brechungsindex und/oder der Transparenz des Materials.

Dieser Effekt unterhalb der Disruptionsschwelle des Materials wird bereits vielfach genutzt, beispielsweise zum Erzeugen von Lichtleitern in Glas ["Micromachining bulk glass by use of femtosecond laser pulses with nanojoule energy", Chris B. Schaffer, André Brodeur, José F. Garcia, and Eric Mazur, Optics Letters, Vol. 26, Issue 2, pp. 93-95], zum Schreiben von 3d-Skulpturen in Glas oder zur Brechungsindexveränderung im Kunststoffmaterial von künstlichen Augenlinsen (siehe DE 10 2004 033 819 A1). Durch die bisherigen Ergebnisse der Untersuchungen an natürlichen Bestandteilen des Auges, insbesondere der Kornea, sah man sich jedoch bestätigt, dass sich durch Einstrahlen von Laserpulsen mit Fluenzen an oder unterhalb der Disruptionsschwelle zumindest mittel- oder langfristig keine Veränderungen der Sehfähigkeit des Patienten ergaben.

Die bekannten Verfahren zur refraktiven Chirurgie leiden nach wie vor in zu vielen Fällen zum Einen an einer mangelnden Vorhersagbarkeit des Ergebnisses, zum Anderen an einem mit Komplikationen verbundenen Verlauf der Wundheilung.

Aufgabe der vorliegenden Erfindung war es, ein Lasersystem und Verfahren zur Korrektur von Sehfehlern zur Verfügung zu stellen, die eine vorteilhafte, insbesondere schneller durchführbare Alternative zu den herkömmlichen Korrekturmöglichkeiten darstellen.

Diese Aufgabe wird gelöst durch ein Lasersystem mit den Merkmalen des Anspruchs 1 bzw. durch ein Verfahren mit den Merkmalen des Anspruchs 12. Vorteilhafte Weiterbildungen der Erfindung sind in den abhängigen Ansprüchen angegeben.

Das erfindungsgemäße Lasersystem zeichnet sich dadurch aus, dass die vom Ultrakurzpulslaser abgegebenen Laserpulse und die Größe des durch die Fokussieroptik festgelegten Brennpunkts (Fokus) so konfiguriert (d.h. aufeinander abgestimmt) sind, dass am Brennpunkt eine Fluenz an oder unterhalb der Disruptionsschwelle des Materials der Augenlinse applizierbar ist, wobei diese Fluenz gleichzeitig hoch genug ist, um Veränderungen einer Materialeigenschaft des Materials der Augenlinse zu bewirken. Die Erfindung beruht auf der Erkenntnis, dass sich durch Applikation von ultrakurzen Laserpulsen an oder unterhalb der Disruptionsschwelle in der Augenlinse dauerhaft bleibende Materialveränderungen erzielen lassen, beispielsweise lokale Veränderungen des Brechungsindex und/oder der Transparenz. Diese Erkenntnis ist vor dem Hintergrund der bisherigen Untersuchungen überraschend, weil in der ebenfalls transparenten Hornhaut (Kornea) zumindest keine dauerhaften Materialveränderungen möglich waren. (Eine denkbare Erklärung wäre ein unterschiedliches Wundheilungsverhalten von Komea und Augenlinse, aber nähere Untersuchungen zu diesen Hintergründen der Erfindung stehen noch aus.) Dass sich mit einer Bearbeitung der Augenlinse Sehfehler korrigieren lassen, hat auch deshalb nicht nahegelegen, weil die Augenlinse im Vergleich zur Komea einen weitaus geringeren Einfluss auf die Gesamt-Brechkraft des Auges hat.

Die Konfiguration bzw. Abstimmung der Laserpulse und der Fokussieroptik bei der Erfindung ist folgendermaßen zu verstehen: Je größer der Winkel (d.h. die Numerische Apertur der Fokussieroptik) ist, unter dem der Laserpuls fokussiert wird, desto geringer kann die Energie eines einzelnen Pulses bei konstanter Pulsdauer sein und desto präziser die Bearbeitung der Augenlinse, ohne dass die Abtragsschwelle des Materials überschritten wird.

Je kürzer hingegen die Laserpulse bei gleicher Numerischer Apertur der Fokussieroptik sind, desto kleiner darf die Pulsenergie sein, um die Abtragsschwelle des Materials nicht zu überschreiten. Die kleinere Pulsenergie führt wiederum dazu, dass die Materialveränderungen auf ein sehr kleines Volumen am Brennpunkt begrenzt bleiben.

Die Wechselwirkung der Pulse des erfindungsgemäßen Lasersystems mit dem Material der Augenlinse erzeugt winzige Läsionen. Kleine Veränderungen (ohne einen Materialabtrag) bleiben am Ort der Wechselwirkung bestehen. Je nach Wahl der Systemparameter können sie Abmessungen von 1-2 Mikrometern oder sogar von weniger als einem Mikrometer haben, beispielsweise von einem oder zwei Zehntel-Mikrometern. Die Wechselwirkung kann durch die Wahl der Lage des Brennpunkts sowohl im Linsenkem der Augenlinse, als auch in oder auf der Linsenrinde erfolgen. Die für die Wechselwirkung an einem Ort erforderliche Fluenz muss dabei nicht mit einem einzigen Laserpuls deponiert werden, sondern sie kann auch durch Bestrahlung derselben Stelle mit einer Vielzahl von Laserpulsen in das Material eingebracht werden.

Das erfindungsgemäße Lasersystem ermöglicht ein einzigartiges, neues Verfahren zur Korrektur von Sehfehlern. Im Gegensatz zu herkömmlichen Verfahren vermeidet es einen Materialabtrag - wodurch gleichzeitig auch die Bildung von Wunden am Auge und alle denkbaren Komplikationen des Wundheilungsverlaufs vermieden werden. Gegenüber den üblichen Verfahren zur refraktiven Chirurgie liegt ein weiterer Vorteil darin, dass mit dem Verfahren nicht die Kornea, sondern die Augenlinse bearbeitet wird. Weil das einfallende Licht bereits durch die Komea gebündelt ist, genügen in der Augenlinse - im Vergleich zur Kornea - kleinere Strukturen, um das Licht zu beeinflussen. Je kleiner die benötigten Strukturen sind, desto schneller - und mit desto geringeren Unannehmlichkeiten für den Patienten - lassen sie sich erzeugen.

Besondere Vorteile ergeben sich, indem die Ablenkeinheit dazu konfiguriert ist, die Brennpunkte einer Gruppe von Laserpulsen so zu setzen, dass durch die Applikation der Laserpulse in der Augenlinse eine diffraktive, d.h. das Licht beugende, optische Struktur erzeugbar ist. Die Läsionen kann man je nach Wahl der Laserparameter so gestalten, dass einfallendes Licht an den Veränderungen gebeugt oder gestreut wird. Erzeugt man eine Vielzahl solcher Läsionen, kann man nach dem Prinzip der diffraktiven Optik bildformende Eigenschaften innerhalb der Linse erzeugen. Mit Hilfe dieser bildformenden Eigenschaften kann man Sehfehler des Auges korrigieren. Beispielsweise kann man durch Erzeugen einer fokussierenden Wirkung die Brechkraft der Linse erhöhen und somit Kurzsichtigkeit korrigieren. Oder man kann durch erzeugen einer defokussierenden Wirkung die Brechkraft der Linse verringern und somit Weitsichtigkeit korrigieren. Außerdem könnte man durch Einbringen einer zylindrischen Wirkung einen Astigmatismus korrigieren. Darüber hinaus ließe sich durch Einbringen einer bifokalen Wirkung die Akkommodation des Auges simulieren und damit die Altersichtigkeit korrigieren.

Bei der diffraktiven optischen Struktur in der Augenlinse könnte es sich um eine zweidimensionale diffraktive Struktur handeln, die im Vergleich zu anderen Strukturen relativ leicht herstellbar wäre. Die Läsionen könnten in einem oder mehreren, jeweils zusammenhängenden "Teppichen" in der Augenlinse platziert werden.

Die zweidimensionale diffraktive Struktur könnte insbesondere eine Mehrzahl von zueinander konzentrischen Ringen oder Ellipsen umfassen, die gemeinsam durch entsprechende Lichtbeugung die Brechkraft der Augenlinse verändern. Ellipsen bieten die Möglichkeit, in unterschiedlichen Raumrichtungen unterschiedliche Brechkraftwirkungen zu erzielen und so z.B. einen Astigmatismus des Auges zu korrigieren.

Alternativ könnte es sich bei der diffraktiven optischen Struktur in der Augenlinse um eine holographische, d.h. dreidimensionale diffraktive Struktur handeln. Diese Möglichkeit bietet sich, weil die Augenlinse bereits ein dreidimensionales Medium zur Aufnahme der holographischen Struktur bereitstellt.

Vorzugsweise ist die Steuereinrichtung des Lasersystems dazu eingerichtet, die Ablenkeinrichtung unter Berücksichtigung des optischen Einflusses der transparenten Komponenten des Patientenauges auf die Laserpulse anzusteuern, insbesondere unter Berücksichtigung des optischen Einflusses der Hornhaut des Auges und der Vorderfläche der Augenlinse. Realisieren lässt sich dies, indem ein digitales Abbild des optischen Systems des Auges im Lasersystem erfasst oder in das Lasersystem eingegeben wird, das dann zur Simulation des Behandlungsergebnisses und/oder zum Erzeugen von Steuerdaten herangezogen wird.

Darüber hinaus ist es vorteilhaft, wenn die Steuereinrichtung dazu eingerichtet ist, die Ablenkeinrichtung anzusteuern unter Berücksichtigung des optischen Einflusses auf einen Laserpuls, der sich aus den Materialveränderungen in der Augenlinse durch die vorangehenden Laserpulse ergibt. Beispielsweise könnten die Laserpulse dazu führen, dass sich das Material der Augenlinse lokal ausdehnt oder zusammenzieht. Diese Formänderung der Augenlinse sollte dann bei der Positionierung der nachfolgenden Laserpulse berücksichtigt werden.

Ideal ist es, wenn die Fokussieroptik eine Numerische Apertur im Bereich von 0,1 bis 1,4 hat, vorzugsweise im Bereich von 0,1 bis 0,3. Mit dieser vergleichsweise starken Fokussierung lassen sich sehr präzise und lokal beschränkte Läsionen bzw. Materialveränderungen erzeugen.

Vorzugsweise hat der Brennpunkt der Fokussieroptik in der Augenlinse einen Durchmesser im Bereich von 0,1 bis 10 Mikrometern, vorzugsweise im Bereich von 0,2 bis 3,0 Mikrometern. Auf diese Weise lassen sich in der Augenlinse diffraktive Strukturen mit einer präzise definierten Geometrie erzeugen.

Die Laserpulse des Lasersystems sollten eine Wellenlänge im Bereich von 400 - 1400 nm haben, vorzugsweise im Bereich von 700 bis 1100 nm, um die Streuung und die Absorption vor der Augenlinse (z.B. in der Kornea) möglichst gering zu halten.

Besonders zweckmäßig sind Laserpulse mit einer Pulsdauer im Bereich von 10 fs bis 1 ps, vorzugsweise im Bereich von 100 - 500 fs. Durch sie lassen sich Läsionen mit einer hohen Präzision erzeugen.

Geeignete Pulsenergien liegen im Bereich von 1 nJ (Nanojoule) bis 10 µJ (Mikrojoule), vorzugsweise im Bereich von 100 nJ bis 3 µJ.

Wenn die Laserpulse eine Pulsfolgefrequenz im Bereich von 1kHz -100 MHz haben, vorzugsweise im Bereich von 10 -1000 kHz, lassen sich innerhalb kurzer Zeit eine Vielzahl von Läsionen setzen, so dass die Behandlung schnell durchführbar ist und für den Patienten mit möglichst geringen Unannehmlichkeiten verbunden ist.

Im Lasersystem kann ein ansteuerbares Verschlusselement zum Festlegen der Pulsfolgefrequenz und/oder der Anzahl der abgegebenen Laserpulse vorgesehen sein. Besonders schnelle Reaktionszeiten können dabei ein akusto-optischer Modulator oder ein elektro-optischer Modulator erzielen. Denkbar wäre aber auch eine ansteuerbare Verschlussblende.

Mit dem erfindungsgemäßen Lasersystem sollte mit einem Laserpuls am Brennpunkt idealerweise eine Fluenz im Bereich von 1 x 10⁻³ J/cm² bis 3,5 x 10⁴ J/cm² erzeugbar sein, vorzugsweise im Bereich von 0,5 J/cm² bis 100 J/cm². Diese Werte haben sich als besonders günstig für eine sub-disruptive Bearbeitung des Augenlinsenmaterials erwiesen.

Um die Laserpulse präzise an die vorbestimmten Orte fokussieren zu können, ist vorzugsweise eine Fixiereinrichtung zum Fixieren der Stellung des Patientenauges relativ zum Lasersystem vorgesehen. Besonders stabil wird die Positionierung des Auges mit einem Saugring. Alternativ könnte auch ein sogenannter "eye-tracker" eingesetzt werden, wenn er eine ausreichende Präzision und Reaktionsgeschwindigkeit gewährleistet.

Die Erfindung bezieht sich auch auf ein Verfahren zum Erzeugen von Steuerdaten zum Ansteuern einer Ablenkeinheit eines ultrakurze Laserpulse erzeugenden, ophthalmologischen Lasersystems, bei dem es sich vorzugsweise um eine der vorstehend beschriebenen Varianten eines Lasersystems handeln kann. Die Steuerdaten umfassen eine Gruppe von Positionssteuerdatensätzen, wobei die Ablenkeinheit mittels eines einzelnen Positionssteuerdatensatzes so ansteuerbar ist, dass eine Fokussiereinrichtung und die Ablenkeinheit in Abhängigkeit von dem Positionssteuerdatensatz die 3-dimensionale Lage eines optischen Brennpunktes von Laserpulsen des Lasersystems in oder auf der Augenlinse eines Patientenauges festlegen. Die Gruppe von Positionssteuerdatensätzen ist so gewählt, dass in der Augenlinse eines Patientenauges eine diffraktive oder holographische Struktur erzeugbar ist, wenn an jedem Brennpunkt mittels mindestens eines ultrakurzen Laserpulses eine Fluenz unterhalb der Abtragsschwelle des Materials der Augenlinse appliziert wird.

Die Steuerdaten könnten im Lasersystem selbst erzeugt werden oder dem Lasersystem drahtlos oder drahtgebunden oder über eine geeignete Schnittstelle in Form einer Datei oder eines Datenstroms zur Verfügung gestellt werden.

Zweckmäßig ist es, wenn die Positionssteuerdaten die zeitliche Abfolge einer Vielzahl von nacheinander an unterschiedlichen Orten erzeugten Brennpunkten festlegen. Diese zeitliche Abfolge könnte dann so gewählt sein, dass die von vorangehenden Laserpulsen erzeugten Läsionen keine Auswirkungen auf nachfolgende Pulse haben, oder dass nebeneinander liegende Läsionen nicht unmittelbar nacheinander erzeugt werden, damit das Material der Augenlinse nach einer Lasereinwirkung mehr Zeit zum Relaxieren hat. Jeder Positionssteuerdatensatz könnte zweidimensionale Koordinaten eines Brennpunktes umfassen, wenn die Lage der Brennpunkte in z-Richtung, d.h. in Richtung der optischen Achse des Auges, durch die Fokussieroptik unveränderlich festgelegt ist. Andernfalls könnte ein Positionssteuerdatensatz auch dreidimensionale Koordinaten umfassen. Die z-Koordinate würde dann zum Ansteuern der Fokussiereinrichtung herangezogen. Die Positionssteuerdaten könnten als kartesische Koordinaten oder als Zylinderkoordinaten dargestellt sein.

Bevorzugt sind die Steuerdaten dazu eingerichtet, die Fokussiereinrichtung und/oder die Ablenkeinrichtung unter Berücksichtigung des optischen Einflusses der transparenten Komponenten des Patientenauges auf die Laserpulse anzusteuern, insbesondere unter Berücksichtigung des optischen Einflusses der Hornhaut des Auges und der Vorderfläche der Augenlinse, um die Brennpunkte präzise an die gewünschten Orte platzieren zu können. Zu diesem Zweck könnte ein Standardmodell eines Auges verwendet werden. Besser ist es jedoch, wenn ein digitales,3-dimensionales, individuelles Modell des zu behandelnden Auges berücksichtigt wird. Dieses digitale Modell wiederum kann patentenangepasst durch bildgebende Verfahren, wie etwa Optische Kohärenztomographie (OCT) oder Ultraschallbildgebung, vor oder während des Eingriffs gewonnen werden. Weist das Lasersystem eine bildgebende Einrichtung auf, könnte diese folglich zur Echtzeit-Überprüfung des Bearbeitungsergebnisses während der Behandlung dienen.

Wie bereits erläutert, könnten die Steuerdaten auch dazu eingerichtet sein, die Ablenkeinrichtung anzusteuern unter Berücksichtigung des optischen Einflusses auf einen Laserpuls, der sich aus den Material- oder Formveränderungen der Augenlinse durch die vorangehenden Laserpulse ergibt.

Zweckmäßig umfassen die Steuerdaten Synchronisationssteuerdaten zum Synchronisieren der Ansteuerung der Ablenkeinheit mit der Abgabe von Laserpulsen von einem Ultrakurzpulslaser, damit die Abgabe der Laserpulse und die jeweilige Positionierung der Brennpunkte ideal aufeinander abgestimmt werden können.

Das Verfahren wird besonders einfach und ist dennoch besonders gut zur Korrektur von Sehfehlern geeignet, wenn die Gruppe von Positionssteuerdaten so gewählt ist, dass die durch die Applikation der Laserpulse erzeugbare diffraktive Struktur zweidimensional ist und eine Mehrzahl von zueinander konzentrischen Ringen oder Ellipsen umfasst. Die Struktur aus konzentrischen Ringen dient dabei zur einheitlichen Veränderung der Brechkraft der Augenlinse, während mit der elliptischen Struktur ein Astigmatismus korrigierbar wäre.

Die diffraktiven Strukturen sollten Abmessungen in der Größenordnung der Wellenlänge des sichtbaren Lichts haben, d.h. in der Größenordnung von ca. 0,4 bis 1 µm, um das einfallende Licht durch Beugung beeinflussen zu können. Auch dreidimensionale Strukturen und andere Strukturen als Ringe oder Ellipsen wären denkbar.

Die Positionssteuerdaten könnten so gewählt sein, dass die durch die Applikation der Laserpulse erzeugbare diffraktive Struktur auf einer ebenen oder auf einer gewölbten Fläche angeordnet ist.

In den meisten Fällen wird es vorteilhaft sein, die Positionssteuerdaten so zu wählen, dass die durch die Applikation der Laserpulse erzeugbare diffraktive Struktur zur optischen Achse des Patientenauges zentriert ist.

Die Erfindung spiegelt sich auch wider in einem Computerprogramm mit Programmcode zum Ausführen einer der vorstehend beschriebenen Verfahrensvarianten, wenn das Computerprogramm auf einem Rechner ausgeführt wird.

Darüber hinaus wird ein refraktiv-Chirurgisches Verfahren zur Behandlung eines Patientenauges beschrieben, bei dem eine Vielzahl von ultrakurzen Laserpulsen an mehreren unterschiedlichen Brennpunkten auf und/oder in der natürlichen Augenlinse des Patientenauges fokussiert werden, wobei mit einem Laserpuls am Brennpunkt eine Fluenz unterhalb der Abtragsschwelle des Materials der Augenlinse appliziert wird, wobei diese Fluenz aber gleichzeitig hoch genug ist, um Veränderungen einer Materialeigenschaft des Materials der Augenlinse zu bewirken, und wobei die Lage der Brennpunkte so gewählt ist, dass in der Augenlinse des Patientenauges durch das Einwirken der fokussierten Laserpulse eine diffraktive optische Struktur erzeugt wird. Neben den vorstehend beschriebenen Verfahrensvarianten könnte die diffraktive Struktur so geformt sein, dass die Augenlinse nach der Behandlung zwei oder mehr verschiedene Brennweiten hat, z.B. durch verschiedene Brechkräfte in unterschiedliche Zonen relativ zur optischen Achse. Auf diese Weise ließe sich einer Alterssichtigkeit (Presbyopie), d.h. einer eingeschränkten Akkomodationsfähigkeit der Augenlinse, entgegenwirken.

Im Folgenden wird ein bevorzugtes Ausführungsbeispiel der Erfindung anhand einer Zeichnung näher erläutert. Im Einzelnen zeigen:
- Fig. 1: ein Ausführungsbeispiel des erfindungsgemäßen Lasersystems in schema- tischer Darstellung,
- Fig. 2: eine Draufsicht auf eine mit dem erfindungsgemäßen Verfahren behandel- te Augenlinse entlang der optischen Achse des Auges.

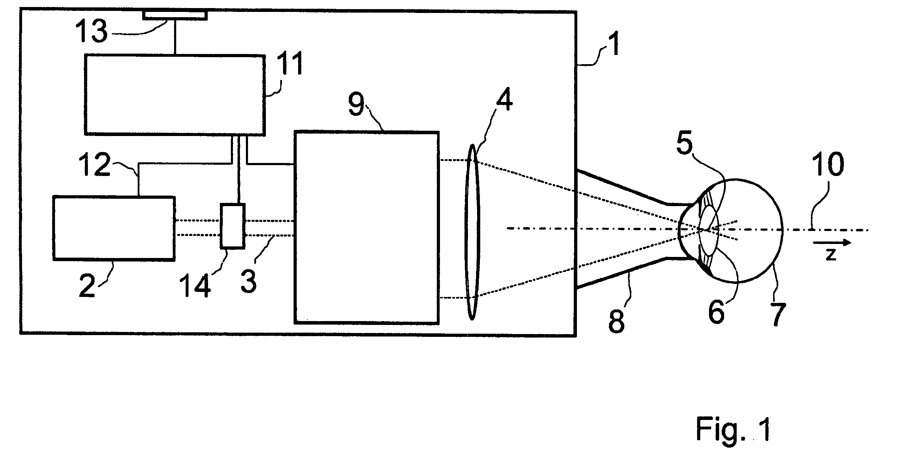

Fig. 1 zeigt in schematischer Darstellung ein Ausführungsbeispiel eines erfindungsgemäßen Lasersystems 1. Das Lasersystem 1 ist insbesondere ein ophthalmologisches Lasersystem, d.h. ein für Augenoperationen geeignetes Lasersystem 1. Es verfügt über einen Laser 2, der Laserstrahlung in Form von ultrakurzen Laserpulsen 3 abgibt. Im bevorzugten Ausführungsbeispiel handelt es sich um einen Femtosekundenlaser 2 mit Pulsdauern im Bereich von einigen Femtosekunden (fs) bis zu einigen 100 fs. Im Interesse möglichst geringen Wartungsaufwandes bieten sich dafür z.B. Faser-Oszillatoren an, mit einer oder ohne eine nachfolgende Verstärkung der Pulse. Typische Werte für die Laserpulse 3 sind eine Pulsdauer von 100 fs, eine Pulsenergie von 1 µJ, eine Wellenlänge von 700 bis 1100 nm und eine Repetitionsrate von 100 kHz.

Eine Fokussieroptik 4 mit einer Numerischen Apertur im Bereich zwischen 0,1 und 1,4, beispielsweise eine einzelne Linse oder ein Linsensystem, fokussiert die Laserpuls 3 auf einen Brennpunkt 5. Die Brennweite der Fokussieroptik 4 ist so gewählt, dass sich der Brennpunkt in oder auf der Augenlinse 6 eines Patientenauges 7 befindet, das mittels einer Fixiereinrichtung 8 in eine vordefinierte, während der Behandlung unverrückbare Stellung relativ zum Lasersystem 1 gebracht ist. Als Fixiereinrichtung 8 kann beispielsweise ein das Auge haltender Saugring verwendet werden. Wahlweise kann anstelle der Fixiereinrichtung eine elektronische, automatische Nachführung des Laserstrahls verwendet werden (ein sog. "Eyetracker"). Die elektronische Nachführung erfasst beispielsweise durch Videobeobachtung die Bewegung des Auges und führt über die Ablenkeinrichtung 9 und die Fokussieroptik 4 den Laserbrennpunkt 5 der Bewegung des Auges 7 nach

Der Brennpunkt 5 hat bevorzugt einen Durchmesser von nur 0,2 bis 1 µm, kann jedoch auch etwas größer sein. Die Numerische Apertur der Fokussieroptik 4 und die Parameter der ultrakurzen Laserpulse 3 sind so aufeinander abgestimmt, dass am Brennpunkt 5 eine Fluenz an oder unterhalb der Disruptionsschwelle des Materials der Augenlinse 6 erzeugbar ist., d.h. beispielsweise von 5 J/cm².

Vor oder hinter der Fokussieroptik 4 ist im Strahlengang des Lasers 2 eine ansteuerbare Ablenkeinrichtung 9 vorgesehen. Als Ablenkeinrichtung 9 eignet sich ein Scannersystem, das in der Regel zwei schwenkbare Spiegel (nicht dargestellt) mit senkrecht zueinander stehenden Schwenkachsen umfasst. Über die Schwenkbewegung der Scannerspiegel ist der Laserstrahl 3 seitlich ablenkbar. Mittels der Ablenkeinrichtung 9 kann die Lage des Brennpunkts 5 der Laserpulse 3 zweidimensional verändert werden, so dass der Brennpunkt 5 an einen beliebigen Punkt auf einer ggf. gewölbten Fläche innerhalb der Augenlinse 6 platziert werden kann.

Auch die Fokussieroptik 4 kann ansteuerbare Elemente umfassen, um die Größe des Brennpunkts 5 und/oder die Lage des Brennpunkts 5 in z-Richtung, d.h. in Richtung der optischen Achse 10 des Auges 7, verändern zu können. In diesem Fall kann die Position des Brennpunkts 5 durch Zusammenwirken der Ansteuerung der Fokussieroptik 4 und der Ablenkeinrichtung 9 sogar dreidimensional auf oder in der Augenlinse 6 variiert werden.

Zur Ansteuerung des Lasers 2, der Fokussieroptik 4 und der Ablenkeinrichtung 9 umfasst das Lasersystem 1 eine Steuereinrichtung 11, beispielsweise einen programmierbaren Mikroprozessor. Die Steuereinrichtung 11 erzeugt Steuerdaten in einem zur Ansteuerung der jeweiligen Komponenten des Lasersystems 1 geeigneten Format. Die Ablenkeinheit 9 erfordert als Steuerdaten beispielsweise Positionsdatensätze, die jeweils die Stellung der beiden Scannerspiegel festlegen.

Über Datenleitungen 12, die die Steuereinrichtung 11 mit dem Laser 2, mit der Ablenkeinrichtung 9 und mit der Fokussieroptik 4 verbinden, kann die Steuereinrichtung 11 die Steuerdaten an alle diese Elemente übermitteln. So kann die Steuereinrichtung 11 beispielsweise für eine Synchronisierung der Ablenkeinrichtung 9 mit der Abgabe der Laserpulse 3 durch den Laser 2 sorgen, um zu verhindern, dass sich die Ablenkeinrichtung 9 gerade beim Eintreffen eines Laserpulses 3 verstellt.

Die Steuereinrichtung 11 verfügt über eine Schnittstelle 13, über die Patientendaten, Messwerte, Befehlsdaten oder andere Daten eingegeben werden können, die anschließend für die Berechnung bzw. Erzeugung der Steuerdaten herangezogen werden. Bei der Schnittstelle 13 kann es sich beispielsweise um ein Laufwerk, eine Tastatur, einen USB-Port und/oder eine drahtlose Schnittstelle handeln.

Als weiteres optisches Element, das ebenfalls durch die Steuereinrichtung 11 ansteuerbar ist, ist im Lasersystem 1 ein Verschlusselement 14 vorgesehen. Im Ausführungsbeispiel ist das Verschlusselement 14 ein akusto-optischer oder elektro-optischer Modulator, da diese Modulatoren eine extrem kurze Reaktionszeit haben und die Laserstrahlung gezielt zwischen zwei Laserpulsen 3 freigeben oder unterbrechen können. Mittels des Verschlusselements 14 kann folglich die Anzahl der abgegebenen Laserpulse 3 festgelegt und ggf. auch die Pulsfolgefrequenz herabgesetzt werden.

Im Folgenden wird das mit dem ophthalmologischen Lasersystem 1 durchführbare Verfahren beschrieben. Wenn keine voreingestellten Standard-Daten verwendet werden, werden zunächst über die Schnittstelle 13 Patientendaten in die Steuereinrichtung 11 eingegeben. Die Patientendaten geben die Abmessungen und/oder die Sehfehler eines Patientenauges 7 wieder. Dabei kann es sich um die Ergebnisse einer vorangehenden Vermessung des Patientenauges 7 handeln.

Aus den verfügbaren Daten berechnet und erzeugt die Steuereinrichtung 11 Steuerdaten. Diese Steuerdaten sind dazu eingerichtet, die Fokussiereinrichtung 4 und/oder die Ablenkeinrichtung 9 unter Berücksichtigung des optischen Einflusses der transparenten Komponenten des Patientenauges 7 auf die Laserpulse anzusteuern, insbesondere unter Berücksichtigung des optischen Einflusses der Hornhaut des Auges und der vorderen Linsenfläche. Zu diesem Zweck könnte die Steuereinrichtung 11 simulieren, wie sich das Sehvermögen des Patienten ändert, wenn in der Augenlinse 6 des Patientenauges 7 eine bestimmte diffraktive optische Struktur erzeugt wird. Auf diese Weise kann die Steuereinrichtung eine zur Korrektur eines oder mehrerer Sehfehler des Patientenauges 7 ideale diffraktive Struktur berechnen. Die ideale diffraktive Struktur ist so gewählt, dass sich durch die Beugung des einfallenden Lichts an ihr die optischen Eigenschaften des Patientenauges 7 so ändem, dass der vorherige Sehfehler möglichst weitgehend aufgehoben ist. Beispielsweise könnte die diffraktive Struktur die Brechkraft des Patientenauges 7 vergrößern oder verkleinern, oder sie könnte verschiedene Zonen mit unterschiedlichen Brechkräften erzeugen. Aus dieser idealen diffraktiven Struktur kann wieder zurückgeschlossen werden auf die Positionen der einzelnen, feinen Läsionen, die in der Augenlinse 6 erzeugt werden müssen, um gemeinsam die ideale diffraktive Struktur zu bilden. Die ideale diffraktive Struktur kann zwei- oder dreidimensional sein.

Anhand der vorstehend beschriebenen Berechnung umfassen die Steuerdaten eine Gruppe von Positionssteuerdatensätzen. Die Ablenkeinheit 9 (und ggf. die Fokussiereinrichtung 4) ist/sind mittels eines einzelnen Positionssteuerdatensatzes so ansteuerbar, dass die Fokussiereinrichtung 4 und die Ablenkeinheit 9 in Abhängigkeit von dem Positionssteuerdatensatz die 3-dimensionale Lage eines optischen Brennpunktes 5 der Laserpulse 3 des Lasersystems 1 festlegen. Wie bereits erläutert, ist die Gruppe von Positionssteuerdatensätzen zudem so gewählt ist, dass in der Augenlinse 6 eines Patientenauges 7 eine diffraktive oder holographische Struktur erzeugbar ist, wenn an jedem Brennpunkt 5 mittels mindestens eines ultrakurzen Laserpulses 3 eine Fluenz unterhalb der Disruptionsschwelle des Materials der Augenlinse 6 appliziert wird. Die Steuerdaten sind zudem dazu eingerichtet, die Ablenkeinrichtung 9 anzusteuern unter Berücksichtigung des optischen Einflusses auf einen Laserpuls 3, der sich aus den Material- oder Formveränderungen der Augenlinse 6 durch die vorangehenden Laserpulse 3 ergibt.

Das zu behandelnde Auge 7 eines Patienten wird mittels der Fixiereinrichtung 8 in eine relativ zum Lasersystem 1 definierte Stellung gebracht und in dieser Stellung festgehalten oder, falls eine automatische Nachverfolgung (Eyetracker) verwendet wird, nachverfolgt. Die Steuerdaten werden von der Steuereinrichtung 11 über die Datenleitungen 12 an den Laser 2, die Fokussieroptik 4, die Ablenkeinheit 9 und das Verschlusselement 14 übermittelt. Eine Vielzahl von Laserpulsen 3 des Lasers 2 wird auf das Patientenauge 7 abgegeben und in oder auf die Augenlinse 6 nacheinander an einer Vielzahl von Brennpunkten 5 fokussiert. Die Lage der einzelnen Brennpunkte 5 der Laserpulse 3 wird durch die Positionssteuerdatensätze bestimmt und vor allem mittels der Ablenkeinheit 9 variiert. An jedem Brennpunkt 5 werden ein oder mehrere Laserpulse 3 appliziert. Die dort deponierte Energiedichte (Fluenz) bewirkt eine Läsion mit einer lokalen Änderung der Materialeigenschaften, vorzugsweise eine lokale Änderung der Transparenz oder des Brechungsindex. Durch die Vielzahl der Läsionen ergibt sich insgesamt eine diffraktive Struktur.

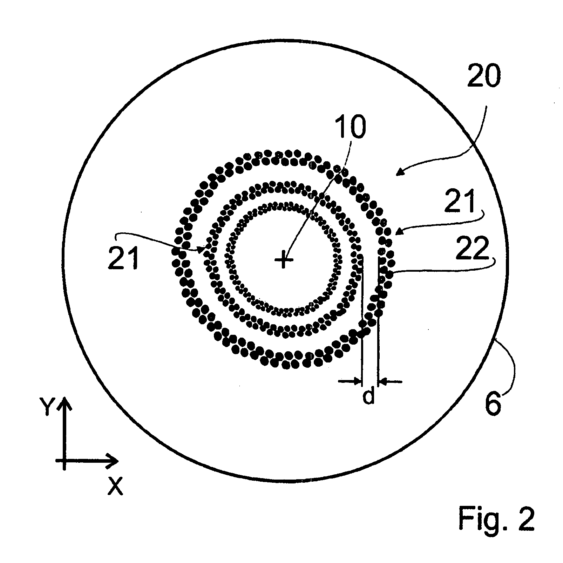

Ein vergleichsweise einfaches Beispiel für eine solche diffraktive Struktur 20 in der behandelten Augenlinse 6 ist in Fig. 2 dargestellt. Fig. 2 ist eine Ansicht des Patientenauges 7 in Richtung der optischen Achse 10 des Auges 7. Die diffraktive Struktur 20 besteht aus mehreren, zueinander und zur optischen Achse 10 konzentrischen Ringen 21, von denen drei Ringe 21 dargestellt sind. Jeder Ring 21 setzt sich zusammen als ein zusammenhängender "Teppich" aus einer Vielzahl von einzelnen, nebeneinander liegenden Läsionen 22 der Augenlinse 6, die jeweils am Ort eines Brennpunkts 5 der Laserstrahlung entstanden sind. Der Ort der einzelnen Läsionen 22 kann in x-y-Koordinaten angegeben werden, denen jeweils ein Positionssteuerdatensatz entspricht.

Der Abstand d zweier Ringe 21 voneinander liegt in der Größenordnung der Wellenlängen des sichtbaren Lichts, kann aber auch etwas größer sein, also z.B. im Bereich von 0,2 µm bis 2,5 µm. Die Läsionen 22 verbleiben dauerhaft (oder zumindest über einen längeren Zeitraum) in der Augenlinse 6. Ebenso dauerhaft kann die diffraktive Struktur 20 daher den Sehfehler des behandelten Auges korrigieren.

In der folgenden Tabelle sind beispielhaft einige für Parameter angegeben, die sich für das Durchführen des erfindungsgemäßen Verfahrens eignen:

| **Parameter** | **Werte für geringen Effekt** | **Werte für starken Effekt** | **Typische Werte (Beispiel 1)** | **Typische Werte (Beispiel 2)** |
|---|---|---|---|---|
| Pulsdauer T [fs] | 10 | 1000 | 100 | 500 |
| Pulsenergie F [nJ] | 1 | 10.000 | 100 | 1000 |
| Mittl. Laserleistung [MW] | 0,1 | 10 | 1 | 2 |
| Brennpunkt-Durchmesser [µm] | 0,2 | 10 | 0,5 | 5 |
| Brennpunktfläche A [cm²] | 3,14 E-10 | 7,85 E-07 | 1,96 E-09 | 1,96 E-07 |
| Intensität I [W/cm²] | 1,27 E+09 | 3,18 E+18 | 5,09 E+14 | 1,02 E+13 |
| Fluenz F [J/cm²] | 1,27 E-03 | 3,18 E+04 | 5,09 E+01 | 5,09 E+00 |

Die "Werte für geringen Effekt" sorgen für eine möglichst geringfügige und auf eine räumlich äußerst kleine Wechselwirkungszone begrenzte Veränderung der Augenlinse 6. Mit diesen Werten lässt sich die Augenlinse sehr präzise bearbeiten; allerdings werden zum Erzeugen größerer Flächen der diffraktiven Struktur 20 evtl. zu viele Läsionen benötigt, was eine entsprechend lange Behandlungszeit bedeutet. Die "Werte für starken Effekt" sorgen für eine großvolumige Materialänderung. Entsprechend weniger Laserpulse werden für eine Behandlung benötigt; allerdings wird das Material der Augenlinse 6 bei den angegebenen Werten relativ stark beansprucht. Typische Werte, die für das Verfahren besonders gut geeignet sind, sind als "Beispiel 1" und "Beispiel 2" angegeben.

Ausgehend von den beschriebenen Ausführungsbeispielen können das erfindungsgemäße Lasersystem und Verfahren in vielfacher Hinsicht abgewandelt werden. Wie erwähnt, kann es sich bei der diffraktiven Struktur 20 auch um eine 3-dimensionale, d.h. holographische Struktur handeln. Denkbar wäre es auch, nicht eine diffraktive, sondern eine refraktive Struktur im Innern der Augenlinse 6 zu erzeugen, d.h. einen "Linsenbereich" mit einer konkaven oder konvexen Grenzfläche und mit einer höheren oder niedrigeren Brechkraft als das natürliche Augenlinsenmaterial.

## Patentansprüche

1. Ophthalmologisches Lasersystem (1) mit
- einem Ultrakurzpulslaser (2) zur Abgabe von ultrakurzen Laserpulsen (3),
- einer Fokussieroptik (4) zum Erzeugen wenigstens eines Brennpunkts (5) auf und/oder in der Augenlinse (6) eines Patientenauges (7),
- einer Ablenkeinrichtung (9) zum Variieren der Position des Brennpunkts (5) auf und/oder in der Augenlinse (6), und
- einer Steuereinrichtung (11) zur Steuerung der Ablenkeinrichtung (9),
**dadurch gekennzeichnet,**
**dass** die vom Ultrakurzpulslaser (2) abgegebenen Laserpulse (3) und die Größe des durch die Fokussieroptik (4) festgelegten Brennpunkts (5) so konfiguriert sind, dass am Brennpunkt (5) eine Fluenz unterhalb der Disruptionsschwelle des Materials der Augenlinse (6) applizierbar ist, wobei diese Fluenz gleichzeitig hoch genug ist, um Veränderungen mindestens einer Materialeigenschaft des Materials der Augenlinse (6) zu bewirken,
und **dass** die Ablenkeinrichtung (9) mittels der Steuereinrichtung (11) derart ansteuerbar ist, dass die Brennpunkte (5) einer Gruppe von Laserpulsen (3) so angeordnet sind, dass durch die mittels der Applikation der Laserpulse (3) bewirkten Veränderungen der Materialeigenschaft in der Augenlinse (6) eine diffraktive optische Struktur (20) erzeugbar ist.

2. Lasersystem nach Anspruch 1, **dadurch gekennzeichnet, dass** die diffraktive optische Struktur (20) in der Augenlinse (6) eine zweidimensionale diffraktive Struktur ist.

3. Lasersystem nach Anspruch 2, **dadurch gekennzeichnet, dass** die zweidimensionale diffraktive Struktur (20) eine Mehrzahl von zueinander konzentrischen Ringen (21) oder Ellipsen umfasst.

4. Lasersystem nach einem der vorangehenden Ansprüche, **dadurch gekennzeichnet, dass** die diffraktive optische Struktur (20) in der Augenlinse (6) eine holographische, dreidimensionale diffraktive Struktur ist.

5. Lasersystem nach einem der vorangehenden Ansprüche, **dadurch gekennzeichnet, dass** die Fokussieroptik (4) eine Numerische Apertur im Bereich von 0,1 bis 1,4 hat, vorzugsweise im Bereich von 0,1 bis 0,3.

6. Lasersystem nach einem der vorangehenden Ansprüche, **dadurch gekennzeichnet, dass** der Brennpunkt (5) der Fokussieroptik (4) in der Augenlinse (6) einen Durchmesser im Bereich von 0,1 bis 10 Mikrometern hat, vorzugsweise im Bereich von 0,2 bis 3,0 Mikrometern.

7. Lasersystem nach einem der vorangehenden Ansprüche, **dadurch gekennzeichnet, dass** die Laserpulse (3) eine Wellenlänge im Bereich von 400 - 1400 nm haben, vorzugsweise im Bereich von 700 bis 1100 nm.

8. Lasersystem nach einem der vorangehenden Ansprüche, **dadurch gekennzeichnet, dass** die Laserpulse (3) eine Pulsdauer im Bereich von 10 fs bis 1 ps haben, vorzugsweise im Bereich von 100 - 500 fs.

9. Lasersystem nach einem der vorangehenden Ansprüche, **dadurch gekennzeichnet, dass** die Laserpulse (3) eine Pulsenergie im Bereich von 1 nJ - 10 µJ haben, vorzugsweise im Bereich von 100 nJ bis 3 µJ.

10. Lasersystem nach einem der vorangehenden Ansprüche, **dadurch gekennzeichnet, dass** die Laserpulse (3) eine Pulsfolgefrequenz im Bereich von 1kHz - 100 MHz haben, vorzugsweise im Bereich von 10 -1000 kHz.

11. Lasersystem nach einem der vorangehenden Ansprüche, **dadurch gekennzeichnet, dass** mit einem Laserpuls (3) am Brennpunkt (5) eine Fluenz im Bereich von 1 x 10⁻³ J/cm² bis 3,5 x 10⁴ J/cm² erzeugbar ist, vorzugsweise im Bereich von 0,5 J/cm² bis 100 J/cm².

12. Verfahren zum Erzeugen von Steuerdaten zum Ansteuern einer Ablenkeinrichtung (9) eines ultrakurze Laserpulse erzeugenden Lasersystems (1),
wobei die Steuerdaten eine Gruppe von Positionssteuerdatensätzen umfassen, wobei die Ablenkeinrichtung (9) mittels eines einzelnen Positionssteuerdatensatzes so ansteuerbar ist, dass eine Fokussiereinrichtung (4) und die Ablenkeinrichtung (9) in Abhängigkeit von dem Positionssteuerdatensatz die 3-dimensionale Lage eines optischen Brennpunktes (5) von Laserpulsen (3) des Lasersystems (1) in oder auf der Augenlinse (6) eines Patientenauges (7) festlegen,
und wobei die Gruppe von Positionssteuerdatensätzen so gewählt ist, dass in der Augenlinse (6) eines Patientenauges (7) eine zweidimensionale oder dreidimensionale diffraktive Struktur (20) erzeugbar ist, wenn an jedem Brennpunkt (5) mittels mindestens eines ultrakurzen Laserpulses (3) eine Fluenz unterhalb der Disruptionsschwelle des Materials der Augenlinse (6) appliziert wird.

13. Verfahren nach Anspruch 12, wobei die Positionssteuerdaten so gewählt sind, dass die durch die Applikation der Laserpulse (3) erzeugbare diffraktive Struktur (20) zweidimensional ist und eine Mehrzahl von zueinander konzentrischen Ringen (21) oder Ellipsen umfasst.

14. Verfahren nach einem der Ansprüche 12 oder 13, wobei die Positionssteuerdaten so gewählt sind, dass die durch die Applikation der Laserpulse (3) erzeugbare diffraktive Struktur (20) auf einer gewölbten Fläche angeordnet ist.

15. Verfahren nach einem der Ansprüche 12 bis 14, wobei die Positionssteuerdaten so gewählt sind, dass die durch die Applikation der Laserpulse (3) erzeugbare diffraktive Struktur (20) zur optischen Achse (10) des Patientenauges (7) zentriert ist.

16. Computerprogramm mit Programmcode zum Ausführen eines Verfahrens nach einem der Ansprüche 12 bis 15, wenn das Computerprogramm auf einem Rechner oder in einer Steuereinrichtung (11) ausgeführt wird.

## Claims

1. Ophthalmologic laser system (1), having
an ultra-short pulse laser (2) for outputting ultra-short laser pulses (3),
a focusing optics (4) for generating at least one focal point (5) on and/or within the eye lens (6) of a patient's eye (7),
a deflection means (9) for varying the position of the focal point (5) on and/or within the eye lens (6), and
a control unit (11) for controlling the deflection means (9),
**characterized in that**
the laser pulses (3) output by the ultra-short pulse laser (2) and the size of the focal point (5) determined by the focusing optics (4) are configured such that a fluence below the disruption threshold of the material of the eye lens (6) can be applied at the focal point (5), said fluence being at the same time sufficiently high to cause changes in at least one material property of the material of the eye lens (6),
and **in that** the deflection means (9) can be actuated by the control unit (11) such that the focal points (5) of a group of laser pulses (3) are arranged such that by the changes in the material property in the eye lens (6) caused by the application of the laser pulses (3), a diffractive optical structure (20) can be generated.

2. Laser system according to claim 1, **characterized in that** the diffractive optical structure (20) in the eye lens (6) is a two-dimensional diffractive structure.

3. Laser system according to claim 2, **characterized in that** the two-dimensional diffractive structure (20) comprises a plurality of rings (21) or ellipses concentric with respect to each other.

4. Laser system according to one of the preceding claims, **characterized in that** the diffractive optical structure (20) in the eye lens (6) is a holographic, three-dimensional diffractive structure.

5. Laser system according to one of the preceding claims, **characterized in that** the focusing optics (4) has a numerical aperture within a range of 0.1 to 1.4, preferably within a range of 0.1 to 0.3.

6. Laser system according to one of the preceding claims, **characterized in that** the focal point (5) of the focusing optics (4) in the eye lens (6) has a diameter within a range of 0.1 to 10 micrometers, preferably within a range of 0.2 to 3.0 micrometers.

7. Laser system according to one of the preceding claims, **characterized in that** the laser pulses (3) have a wavelength within a range of 400 to 1400 nm, preferably within a range of 700 to 1100 nm.

8. Laser system according to one of the preceding claims, **characterized in that** the laser pulses (3) have a pulse duration within a range of 10 fs to 1 ps, preferably within a range of 100 to 500 fs.

9. Laser system according to one of the preceding claims, **characterized in that** the laser pulses (3) have a pulse energy within a range of 1 nJ to 10 µJ, preferably within a range of 100 nJ to 3 µJ.

10. Laser system according to one of the preceding claims, **characterized in that** the laser pulses (3) have a pulse repetition rate within a range of 1 kHz to 100 MHz, preferably within a range of 10 to 1000 kHz.

11. Laser system according to one of the preceding claims, **characterized in that** a fluence within a range of 1 x 10⁻³ J/cm² to 3.5 x 10⁴ J/cm², preferably within a range of 0.5 J/cm² to 100 J/cm², can be generated at the focal point (5) with a laser pulse (3).

12. Method for generating control data for actuating deflection means (9) of an ultra-short laser pulse generating laser system (1),
wherein the control data comprise a group of position control data records, where the deflection means (9) can be actuated by means of one single position control data record, such that a focusing means (4) and the deflection means (9) determine the three-dimensional position of an optical focal point (5) of laser pulses (3) of the laser system (1) within or on the eye lens (6) of a patient's eye (7), depending on the position control data record,
and wherein the group of position control data records is selected such that a two-dimensional or three-dimensional diffractive structure (20) can be generated in the eye lens (6) of a patients' eye (7), if a fluence below the disruption threshold of the material of the eye lens (6) is applied at each focal point (5) by means of at least one ultra-short laser pulse (3).

13. Method according to claim 12, wherein the position control data are selected such that the diffractive structure (20) that can be generated by the application of the laser pulses (3) is two-dimensional and comprises a plurality of rings (21) or ellipses concentric with respect to each other.

14. Method according to one of claims 12 or 13, wherein the position control data are selected such that the diffractive structure (20) that can be generated by the application of the laser pulses (3) is arranged on a curved surface.

15. Method according to one of claims 12 to 14, wherein the position control data are selected such that the diffractive structure (20) that can be generated by the application of the laser pulses (3) is centered with respect to the optical axis (10) of the patient's eye (7).

16. Computer program with program code for carrying out a method according to one of claims 12 to 15, if the computer program is run on a computer or in the control unit (11).

## Revendications

1. Système laser ophtalmologique (1) avec
- un laser à impulsions ultracourtes (2) pour émettre des impulsions lasers ultracourtes (3),
- une optique de focalisation (4) pour produire au moins un point focal (5) sur et/ou dans la lentille oculaire (6) de l'oeil (7) d'un patient,
- un dispositif de déviation (9) pour varier la position du point focal (5) sur et/ou dans la lentille oculaire (6), et
- un dispositif de commande (11) pour commander le dispositif de déviation (9),
**caractérisé en ce que** les impulsions lasers (3) émises par le laser à impulsions ultracourtes (2) et la taille du point focal (5) fixé par l'optique de focalisation (4) sont conçus de telle sorte qu'au niveau du point focal (5), une fluence inférieure au seuil de disruption de la matière de la lentille oculaire (6) soit applicable, étant précisé que cette fluence est en même temps suffisamment élevée pour provoquer des modifications d'au moins une propriété de la matière de la lentille oculaire (6),
et **en ce que** le dispositif de déviation (9) est apte à être commandé à l'aide du dispositif de commande (11) pour que les points focaux (5) d'un groupe d'impulsions lasers (3) soient disposés de telle sorte qu'une structure optique diffractive (20) puisse être produite dans la lentille oculaire (6) par les modifications des propriétés de matériau provoquées à l'aide de l'application des impulsions lasers (3).

2. Système laser selon la revendication 1, **caractérisé en ce que** la structure optique diffractive (20) dans la lentille oculaire (6) est une structure diffractive bidimensionnelle.

3. Système laser selon la revendication 2, **caractérisé en ce que** la structure diffractive bidimensionnelle (20) comprend une multiplicité d'anneaux (21) ou d'ellipses concentriques.

4. Système laser selon l'une des revendications précédentes, **caractérisé en ce que** la structure optique diffractive (20) dans la lentille oculaire (6) est une structure diffractive tridimensionnelle holographique.

5. Système laser selon l'une des revendications précédentes, **caractérisé en ce que** l'optique de focalisation (4) a une ouverture numérique située dans la plage de 0,1 à 1,4, de préférence dans la plage de 0,1 à 0,3.

6. Système laser selon l'une des revendications précédentes, **caractérisé en ce que** le point focal (5) de l'optique de focalisation (4) dans la lentille oculaire (6) a un diamètre situé dans la plage de 0,1 à 10 microns, de préférence dans la plage de 0,2 à 3,0 microns.

7. Système laser selon l'une des revendications précédentes, **caractérisé en ce que** les impulsions lasers (3) ont une longueur d'onde située dans la plage de 400-1400 nm, de préférence dans la plage de 700 à 1100 nm.

8. Système laser selon l'une des revendications précédentes, **caractérisé en ce que** les impulsions lasers (3) ont une durée d'impulsion située dans la plage de 10 fs à 1 ps, de préférence dans la plage de 100-500 fs.

9. Système laser selon l'une des revendications précédentes, **caractérisé en ce que** les impulsions lasers (3) ont une énergie d'impulsion située dans la plage de 1 nJ-10 µJ, de préférence dans la plage de 100 nJ à 3 µJ.

10. Système laser selon l'une des revendications précédentes, **caractérisé en ce que** les impulsions lasers (3) ont une fréquence de succession d'impulsions située dans la plage de 1 kHz-100 MHz, de préférence dans la plage de 10-1000 kHz.

11. Système laser selon l'une des revendications précédentes, **caractérisé en ce qu'**avec une impulsion laser (3) au niveau du point focal (5) une fluence située dans la plage de 1x10⁻³ J/cm² à 3,5x10⁴ J/cm², de préférence dans la plage de 0,5 J/cm² à 100 J/cm² peut être produite.

12. Procédé pour produire des données de commande pour commander un dispositif de déviation (9) d'un système laser (1) produisant des impulsions lasers ultracourtes,
étant précisé que les données de commande comprennent un groupe de jeux de données de commande de position, que le dispositif de déviation (9) est apte à être commandé à l'aide d'un jeu individuel de données de commande de position de telle sorte qu'un dispositif de focalisation (4) et le dispositif de déviation (9) fixent en fonction du jeu de données de commande de position la position en trois dimensions d'un point focal optique (5) d'impulsions lasers (3) du système laser (1) dans ou sur la lentille oculaire (6) de l'oeil (7) d'un patient,
et que le groupe de jeux de données de commande de position est choisi pour qu'une structure diffractive (20) à deux ou trois dimensions puisse être produite dans la lentille oculaire (6) de l'oeil (7) d'un patient si une fluence inférieure au seuil de disruption de la matière de la lentille oculaire (6) est appliquée au niveau de chaque point focal (5) à l'aide d'au moins une impulsion laser ultracourte (3).

13. Procédé selon la revendication 12, étant précisé que les données de commande de position sont choisies pour que la structure diffractive (20) apte à être produite par l'application des impulsions lasers (3) soit bidimensionnelle et comprenne une multiplicité d'anneaux (21) ou d'ellipses concentriques.

14. Procédé selon l'une des revendications 12 ou 13, étant précisé que les données de commande de position sont choisies pour que la structure diffractive (20) apte à être produite par l'application des impulsions lasers (3) soit disposée sur une surface bombée.

15. Procédé selon l'une des revendications 12 à 14, étant précisé que les données de commande de position sont choisies pour que la structure diffractive (20) apte à être produite par l'application des impulsions lasers (3) soit centrée par rapport à l'axe optique (10) de l'oeil (7) du patient (7).

16. Programme informatique avec un code de programme pour la mise en oeuvre d'un procédé selon l'une des revendications 12 à 15 si le programme informatique est exécuté sur un ordinateur ou dans un dispositif de commande (11).
